# EUROPEAN PATENT SPECIFICATION

(11) **EP 2 858 703 B1**
(45) Date of publication and mention of the grant of the patent: **23.11.2016**
(21) Application number: 13739287.4
(22) Date of filing: 28.05.2013
(51) Int. Cl.: A61M 16/06

(54) **PEDIATRIC TOTAL FACEMASK**
KOMPLETTE PÄDIATRISCHE GESICHTSMASKE
MASQUE FACIAL PÉDIATRIQUE INTÉGRAL

(30) Priority: 07.06.2012 US 201261656737 P
(43) Date of publication of application: 15.04.2015
(73) Proprietor: Koninklijke Philips N.V., 5656 AE Eindhoven (NL)
(72) Inventor: MCCRACKEN, Christopher, James, NL-5656 AE Eindhoven (NL)
(74) Representative: Steffen, Thomas
(86) International application number: PCT/IB2013/054390
(87) International publication number: WO 2013/182952

(56) References cited:
- US-A- 5 924 420
- US-A1- 2003 145 859
- US-A1- 2010 258 133
- US-A1- 2012 037 161

## Description

### BACKGROUND OF THE INVENTION

### 1. Field of the Invention

The present invention relates to therapeutic gas delivery systems and, more particularly, to a mask that forms a seal with a pediatric patient's face during gas delivery.

### 2. Description of the Related Art

There are numerous situations where it is necessary or desirable to deliver a flow of breathing gas non-invasively to the airway of a patient, i.e., without intubating the patient or surgically inserting a tracheal tube in their esophagus. For example, it is known to ventilate a patient using a technique known as non-invasive ventilation (NIV). It is also known to deliver continuous positive airway pressure (CPAP) or variable airway pressure, which varies with the patient's respiratory cycle, to treat a medical disorder, such as sleep apnea syndrome, in particular, obstructive sleep apnea (OSA), chronic obstructive pulmonary disease (COPD), or congestive heart failure (CHF).

Non-invasive ventilation and pressure support therapies involve the placement of a patient interface device, which is typically a nasal or nasal/oral mask (i.e., a full face mask), on the face of a patient to interface the ventilator or pressure support system with the airway of the patient so that a flow of breathing gas can be delivered from the pressure/flow generating device to the airway of the patient.

One class of respiratory face mask assemblies can be of two different types: a single limb circuit type and a dual limb circuit type. For a single limb circuit, the face mask assembly typically includes a valve and an exhaust port, and, for a dual limb circuit, the face mask assembly typically does not include a valve but provides a valveless conduit instead. Other types of masks may also be useful for different applications. Thus, hospitals and other health care facilities typically stock several different types of face mask assemblies that are used for different applications. Cost and storage space considerations associated with stocking several different face mask assemblies can be significant.

A particular segment of the population which for which hospitals generally donot stock dedicated masks are pediatric patients. One reason masks for such patients are not stocked is the small demand. A bigger reason, is a lack of masks available for use on pediatric patients, particularly for the smallest of such patients.

US 2010/258133 A1 discloses a mask assembly having a mask body which may be in form of a full-face mask. The mask body has an opening for receiving a treatment gas supply and a flexible peripheral seal structure having a first side coupled to the mask body and an opposite second side including an opening for receiving the face of a patient.

US 2003/0145859 A1 discloses a full-face mask and head gear system for improving pediatric compliance of ventilation therapy. A nasal mask being described as having a height to width ratio of 0.87. Full-face masks have all height to width ratios larger than 1.

US 2012/0037161 A1 discloses a mask system fitting to pre-adult patients. Full masks maybe provided having a cushion which may include a double-walled face contacting portion. A comparison of the sizes of different cushions is shown. Absolute sizes of the opening for receiving a patient's face are not mentioned.

Accordingly, a need exists for a patient interface assemblies that improve upon existing assemblies, for example, to maximize patient comfort while minimizing leak, during delivery of a positive airway pressure or flow of gas to the airway of a pediatric patient.

### SUMMARY OF THE INVENTION

As one aspect of the invention a mask assembly for use in a system delivering a flow of a treatment gas to the airway of a pediatric patient is provided. The mask assembly comprises: a mask body having an opening adapted to receive a treatment gas supply and a flexible peripheral seal structure having a first side coupled to the mask body and an opposite second side including an opening adapted to receive the face of the pediatric patient such that the second side sealingly engages the perimeter of the pediatric patient's face. The opening is defined by a height and a width, and the ratio of the height to width of the opening is in the range of about 0.83 to about 0.87. In one embodiment, the opening may have a height of about 60.5mm and a width of about 73mm.

The pediatric mask assembly may further comprise a headgear assembly selectively coupled to the mask body. The headgear assembly may comprise a bonnet-style headgear assembly. The headgear assembly may comprise a color indicative of the gender of the pediatric patient for which the assembly is intended. The headgear assembly may be one of: a shade of the color blue to indicate a male pediatric patient or a shade of the color pink to indicate a female patient.

As another aspect of the invention, a system for delivering treatment gas to the airway of a pediatric patient is provided. The system comprises a pressure/flow generating system, a tubing element having a first end coupled to the pressure/flow generating system and an opposite second end, a mask assembly as previously discussed, and a headgear assembly selectively coupled to the mask body.

As yet another aspect of the invention, a kit is provided. The kit comprises a mask assembly, as previously described, and a plurality of headgear assemblies, each headgear assembly being selectively coupled to the mask assembly. At least one headgear assembly comprises a color adapted to indicate a male patient and at least another one headgear assembly comprises a color adapted to indicate a female patient.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1A is a perspective view of a system including a mask assembly disposed on a pediatric patient's face in accordance with an embodiment of the present invention;
FIG. 1B is a left side perspective view of the system and mask assembly of FIG. 1A;
FIG. 2 is a perspective view of the mask assembly of FIGS. 1A and 1B coupled to an entrainment valve assembly in accordance with an embodiment of the present invention;
FIG. 3 is a perspective exploded view of a mask assembly in accordance with an embodiment of the present invention;
FIG. 4 is a perspective view of an air entrainment valve with exhaust port assembly in accordance with an embodiment of the present invention;
FIG. 5 is another perspective view of the air entrainment valve with exhaust port assembly of FIG. 4;
FIG. 6 is a cross-sectional view of the mask assembly of FIG. 2;
FIG. 7 is a cross-sectional view of the entrainment valve assembly and breathing circuit interface of the mask assembly of FIG. 2;
FIG. 8 is an elevational view of the patient side of the mask assembly of FIG. 2; and
FIG. 9 is a perspective view of the lower patient side of the mask assembly of FIG. 2.

### DETAILED DESCRIPTION OF THE EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, the phrase that two or more elements are "selectively coupled" shall mean the elements are coupled in a manner that may be readily positioned in either of a coupled or uncoupled position.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Mask embodiments designed in accordance with the present invention are specifically designed for the particular facial shape/structure of a young child from birth to 18 months, hence the phrase "pediatric patient" shall mean a patient that is 18 months old or younger. Such young children typically display a facial shape/structure having a facial height to width ratio in the range of about 0.83 to about 0.87. Such facial shape/structure is in contrast to a typical child/adult facial shape/structure which has a height to width ratio of 1.0. Accordingly, conventional full-face masks suitable for children and adults will not work for the intended pediatric patients to which embodiments of the present invention are directed.

Directional phrases used herein, such as, for example and without limitation, top, bottom, left, right, upper, lower, front, back, and derivatives thereof, relate to the orientation of the elements shown in the drawings and are not intended to be limiting upon the claims unless expressly recited therein.

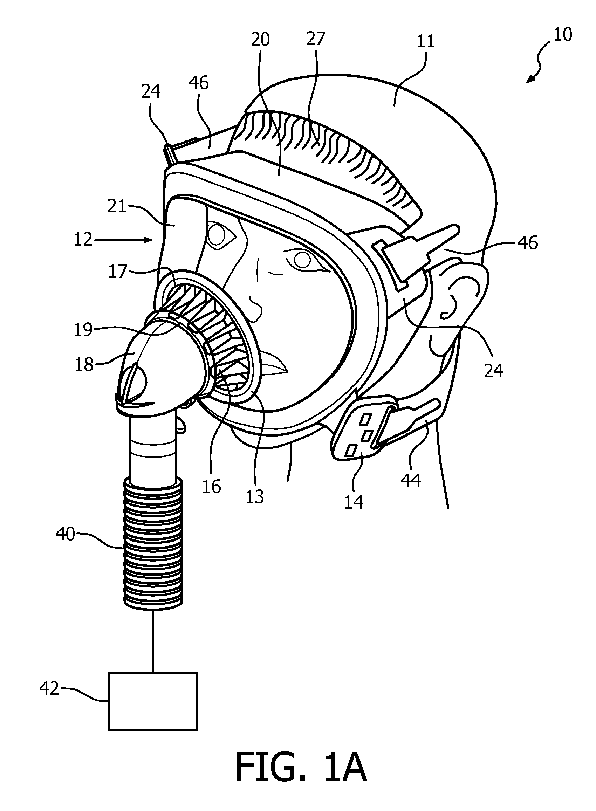

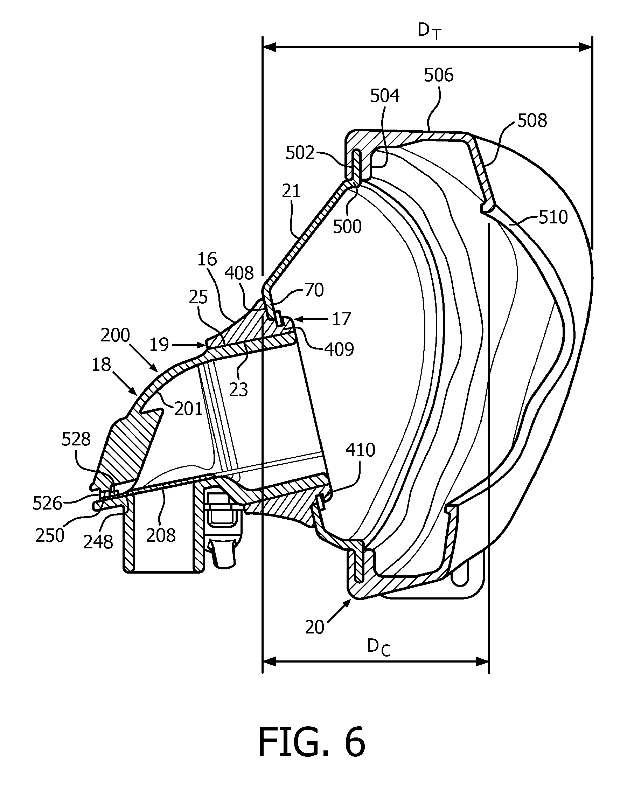

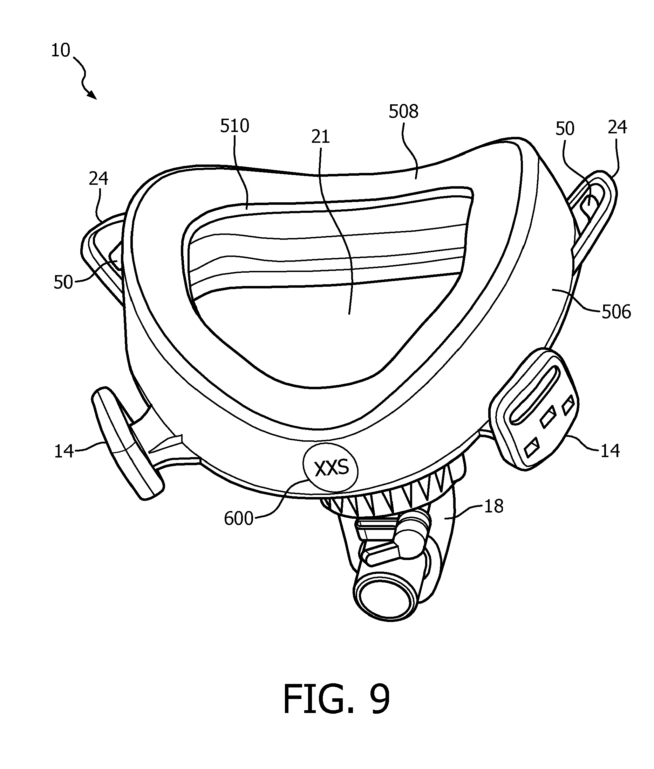

FIGS. 1A, 1B and 2-9 show a mask assembly 10 and related components for use in therapeutic gas delivery in accordance with an embodiment of the present invention. Referring to FIGS. 1A, 1B, 2 and 3, mask assembly 10 may generally include a mask body 12 having an opening 13 for reception of a breathing gas supply. Mask body 12 includes a seal structure 20 for sealingly engaging with the face of a pediatric patient 27 (FIG. 1A) in surrounding relation to at least the nose and mouth (and optionally the eyes) of pediatric patient 27. As shown in FIG. 6, mask assembly 10 has a total depth D_{T} at outer edges in an area that would generally coincide with a pediatric patient's temples and a central depth D_{C} that is less than total depth D_{T} due to the generally curved shape of seal structure 20, as perhaps best shown in FIG. 9. In an example embodiment, mask assembly 10 has a total depth D_{T} of about 67mm and a central depth D_{C} of about 48mm.

Mask assembly 10, in one embodiment, also includes a breathing circuit interface 16 for connecting mask body 12 with a pressurized breathing gas supply. As disclosed in more detail below, breathing circuit interface 16 has a first portion 17 rotatably connected with mask body 12 and a second portion 19 constructed and arranged to connect with a conduit 18 for delivering the breathing gas supply to pediatric patient 27 through opening 13.

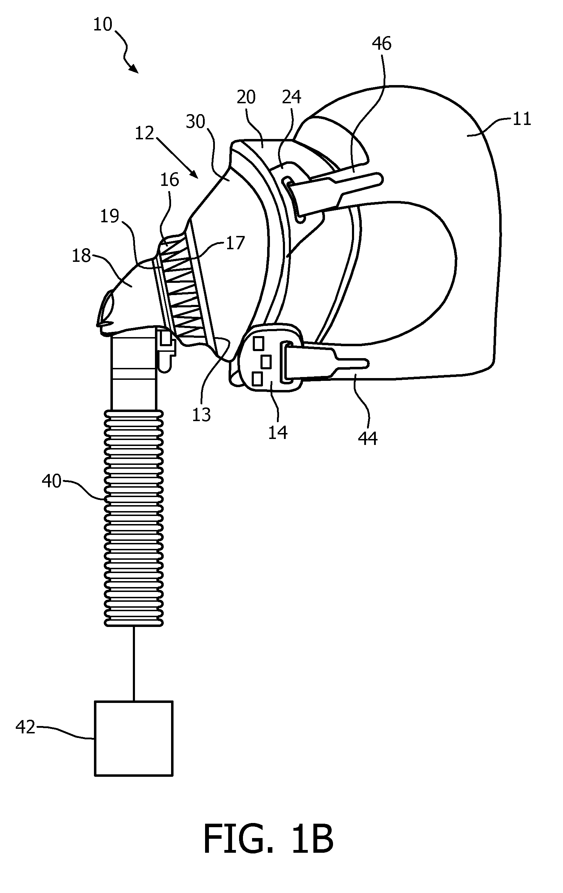

In an example embodiment, breathing circuit interface 16 and conduit 18 connect mask body 12, via a circuit tubing 40, to a pressure/flow generating system 42 (shown schematically in FIGS. 1A and 1B), such as a ventilator, CPAP device, or variable pressure device, e.g., a BiPAP^{®} positive pressure therapy device manufactured and distributed by Philips Respironics, Inc. of Pittsburgh, Pa., or an auto-titration pressure support system. Such combination of elements is typically referred to as a "system" for delivering treatment gas to a patient.

A BiPAP device is a bi-level device in which the pressure provided to the patient varies with the patient's respiratory cycle, so that a higher pressure is delivered during inspiration than during expiration. An auto-titration pressure support system is a system in which the pressure varies with the condition of the patient, such as whether the patient is snoring or experiencing an apnea or hypopnea. For present purposes, pressure/flow generating system 42 is also referred to as a gas flow generating device, because gas flow results when a pressure gradient is generated. The present invention contemplates that pressure/flow generating system 42 is any conventional system for delivering a flow of gas to an airway of a patient or for elevating a pressure of gas at an airway of the patient, including the pressure support systems summarized above and non-invasive ventilation systems.

As will be appreciated from further discussions herein, second portion 19 of breathing circuit interface 16 is releasably connected with conduit 18 to enable different types of conduits 18 to be connected to mask body 12. Accordingly, it is to be appreciated that other conduits beyond those described in detail herein may be employed with mask body 12 without varying from the scope of the present invention. In addition, a rotatable or swivel connection between breathing circuit interface 16 at first portion 17 thereof with mask body 12 allows elbow shaped conduit 18 to rotate after connection to enable conduit 18 to extend in any direction within 360° of rotation for connecting with tubing 40. It should be appreciated that for some purposes breathing circuit interface 16 may also be considered to be part of mask body 12.

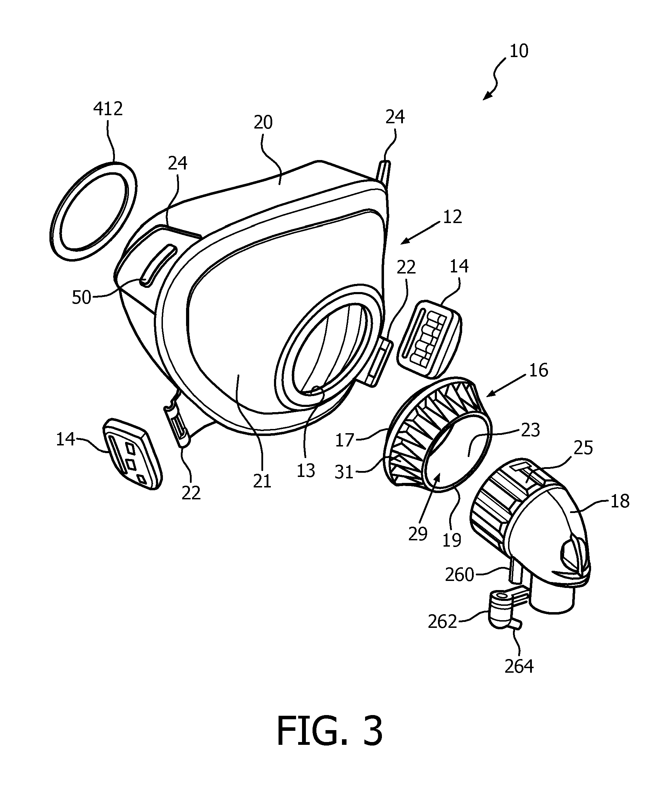

As shown in FIG. 3, breathing circuit interface 16 has an annular configuration with a generally cylindrical inner surface 23 disposed about a central opening, shown generally at 29, therethrough. As will be appreciated from more detailed discussions later, cylindrical inner surface 23 of breathing circuit interface 16 is shaped and configured to provide a releasable friction fit with a generally cylindrical mating surface 25 of an appropriate conduit 18 that connects with tubing 40 for receiving a breathable gas.

In the exemplary embodiment, such as that shown in FIG. 3, breathing circuit interface 16 includes a plurality of radially outwardly extending ribs 31 spaced at regular circumferential intervals about the outer surface (not numbered) thereof. Each of ribs 31 have an increasing thickness or radial dimension as they extend from second portion 19 to first portion 17 of breathing circuit interface 16. Ribs 31 are integrally formed as part of the outer surface of breathing circuit interface 16. The plurality of ribs 31 located on the outer surface of second portion 19 of breathing circuit interface 16 provides healthcare personnel a grip to hold breathing circuit interface 16 when connecting and disconnecting conduit 18 to breathing circuit interface 16. Ribs 31 also facilitate manual rotation of breathing circuit interface 16. The present invention contemplates that any technique for coupling the breathing circuit to the mask body can be used in mask assembly 10.

In one example embodiment, mask body 12 includes a rigid portion 21, formed from a clear plastic material, and the aforementioned flexible peripheral seal structure 20. Flexible peripheral seal structure 20 is attached around rigid portion 21 of mask body 12.

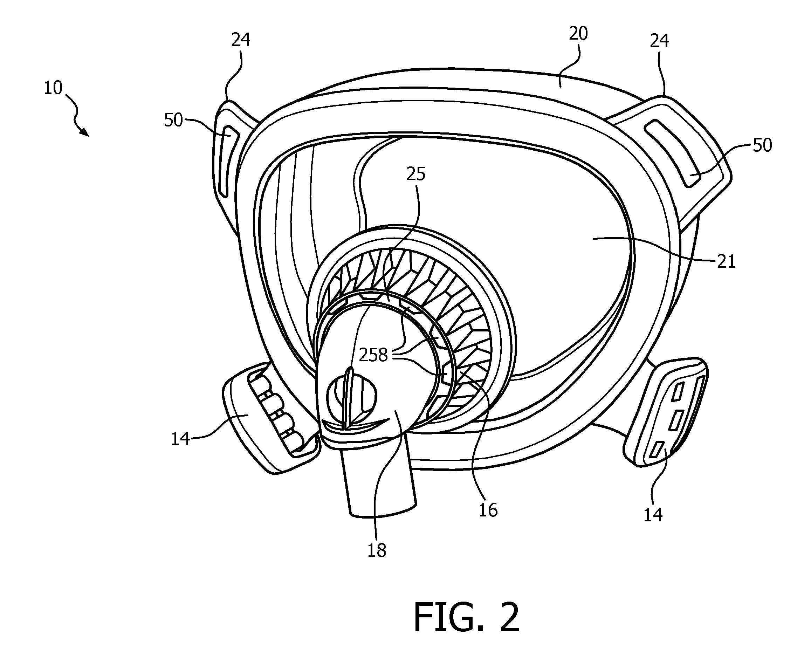

In one example embodiment, such as shown in FIGS. 1A and 1B, mask body 12 is adapted to be connected with a headgear assembly 11 that can be used to mount mask body 12 on the head of pediatric patient 27. In an example embodiment, a pair of headgear attachment clips 14 are provided for interface and connection with lower headgear mounting strap portions 44 of headgear assembly 11. A pair of headgear attachment members 22 (FIG. 3) is provided for connectably receiving headgear attachment clips 14, and a pair of spaced upper headgear strap retaining tabs 24, each having an elongated opening 50 therethrough, is provided for receiving upper headgear mounting strap portions 46 of headgear assembly 11. The pair of headgear retaining tabs 24 is disposed on the opposite upper sides of rigid portion 21 of mask body 12. The pair of headgear attachment members 22 is disposed on opposite, lower sides of rigid portion 21 of mask body 12. Each headgear retaining tab 24 is integrally formed with rigid portion 21 and extends outwardly from flexible peripheral seal structure 20, as perhaps best seen in FIGS. 2 and 3.

In an example embodiment, mask assembly 10 is characterized by mask body 12 having a weight of 20.27 grams (g), seal structure 20 having a weight of 33.72g, mask assembly 10 along with breathing circuit interface 16 and elbow having a weight (no headgear) of 98.24g, and mask assembly 10 with headgear having a weight of 124.10g.

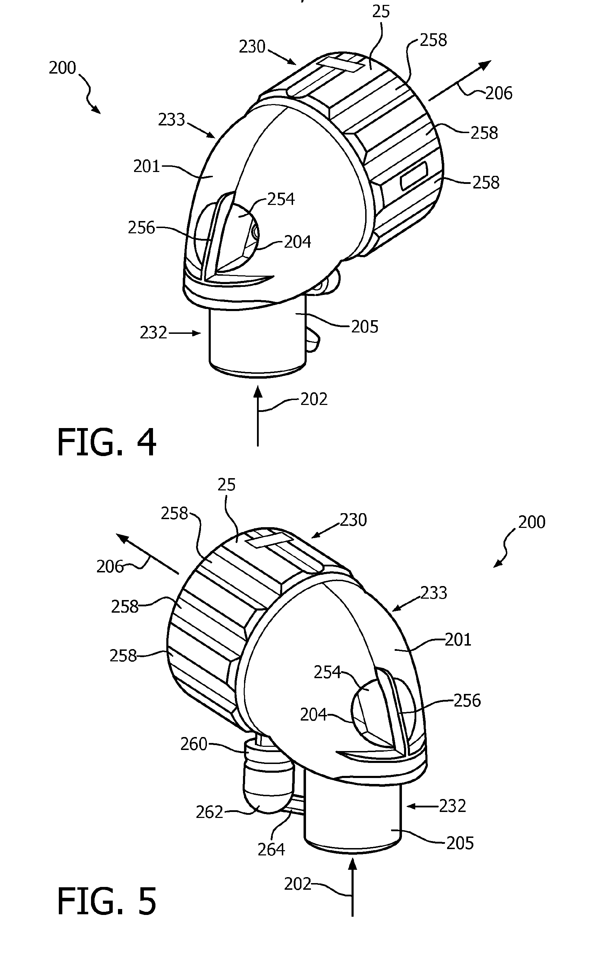

FIG. 4 shows a conduit 18 in accordance with one embodiment. In this embodiment, conduit 18 is an entrainment valve assembly 200. Entrainment valve assembly 200 comprises a generally elbow shaped tubular member 201 formed from a rigid plastic material, such as polycarbonate or other plastic material as would be appreciated by one skilled in the art. In one example embodiment, tubular member 201 is formed from a clear, colorless, plastic material. In another example embodiment, tubular member 201 is formed from an orange/amber colored material, however members of other colors and/or opacity may be employed without varying from the scope of the present invention. Tubular member 201 includes a primary inlet 202, a secondary inlet 204 and an outlet 206.

Tubular member 201 includes a first connector portion 230 and a second connector portion 232. First connector portion 230 and second connector portion 232 are generally cylindrical in shape and are generally disposed perpendicular to each other. First connector portion 230 and second connector portion 232 are joined by a bent tubular region 233. First connector portion 230 has aforementioned generally cylindrical outer surface 25 for connection with breathing circuit interface 16, while second connector portion 232 also has a cylindrical outer surface 205 for frictionally mating with inner surfaces of tubing 40 for receiving pressurized gas from a pressure/flow generating system 42.

Secondary inlet 204 of entrainment valve assembly 200 comprises an opening 254 located towards bent tubular region 233. Opening 254 is divided into two equal, generally semi-cylindrical segments by a planar wall 256. Planar wall 256 of entrainment valve assembly 200 extends through cylindrical opening 254. Opening 254 allows pediatric patient 27 to breath in from and out to atmosphere in the absence of a pressurized gas flow being provided into inlet 202. Entrainment valve assembly 200, at cylindrical surface 25, further includes a plurality of exhalation grooves 258. Grooves 258 are located at an interface where entrainment valve assembly 200 connects with breathing circuit interface 16. The plurality of exhalation grooves 258 are circumferentially spaced on surface 25 and placed symmetrically on either side of first connector portion 230. Other example embodiments are contemplated in which exhalation grooves 258 are located anywhere on the outer surface of first connector portion 230, where it interfaces with breathing circuit interface 16.

Exhalation grooves 258 are sufficiently long so that, when entrainment valve assembly 200 is pushed as far as it can go into breathing circuit interface 16 (such as shown in FIGS. 1A, 1B and 2), grooves 258 still extend outwardly from breathing circuit interface 16 and provide a path for allowing gases exhaled from the pediatric patient to exit through grooves 258. In addition, for any extent of friction fitting engagement between surfaces 23 and 25, the cross-sectional area of the gap or space provided by grooves 258 will be constant, so that the expired gas flow path to the exterior of mask body 12 provides constant resistance, irrespective of whether entrainment valve assembly 200 is fully inserted or somewhat less than fully inserted into breathing circuit interface 16.

Referring to FIGS. 3 and 5, entrainment valve assembly 200 includes a pressure port 260 which extends from bent tubular region 233 of entrainment valve assembly 200 and is generally parallel to second connector portion 232 of entrainment valve assembly 200. A removable cap 262 is used to close pressure port 260. Cap 262 includes a gripping tab 264 to aid in removal of cap 262 from pressure port 260. A sampling tube (not shown) may be disposed in fluid communication with the gas within tubular body 201 via pressure port 260. A transducer (not shown) can be secured to the sampling tube in conjunction with a processor which communicates with the transducer. The processor calculates at least one respiratory parameter using the signal from the transducer. Such arrangement is generally used to measure pressure by the ventilator as control feedback to the ventilator.

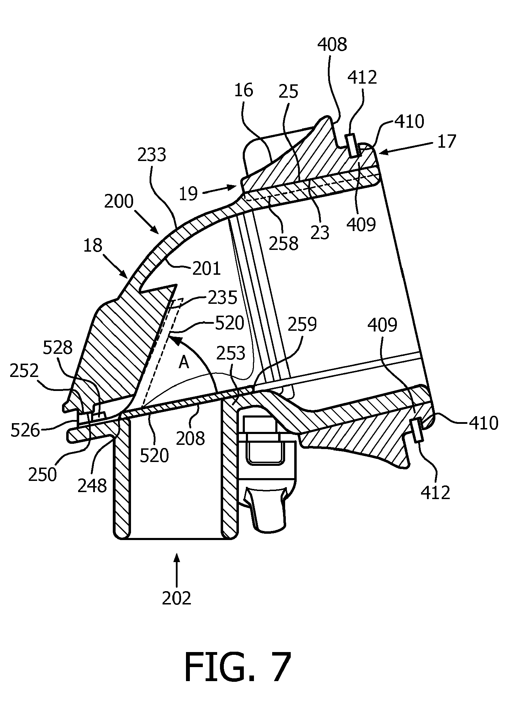

As shown in FIGS. 6 and 7, breathing circuit interface 16 includes the aforementioned first portion 17 and second portion 19. First portion 17 is generally circular in shape and includes an annular flat wall 408 that engages a radially inwardly extending flange portion 70 in slidable surface relationship. Flange portion 70 surrounds opening 13 in rigid portion 21 of mask body 12 (see FIG. 3). First portion 17 of breathing circuit interface 16 further includes a generally cylindrical protruding portion 409 that extends outwardly from a radially innermost portion of annular surface 408. Cylindrical protruding portion 409 extends into opening 13 in rigid portion 21 of mask body 12. Cylindrical protruding portion 409 includes a groove 410 located in the outer cylindrical surface thereof (see FIG. 7). Groove 410 accommodates a connecting washer or a bearing 412. Washer 412 in one embodiment is a split ring washer structure that has an outer periphery thereof that bears against the inner surface of flange 70, and its inner periphery received in groove 410 so as to rotatably connect breathing circuit interface 16 with mask body 12. Thus, breathing circuit interface 16 is rotatably connected with rigid portion 21 of mask body 12. Slight friction at the rotatable interface may, in one embodiment, provide at least resistance to rotation, so that the rotational position of breathing circuit interface 16 can be manually set as desired, and it will retain that position so that the leg or second connector portion 232 of conduit 18 that connects with tubing 40 can be positioned in a desired direction that is generally retained unless intentionally altered. In another embodiment, the friction at the point of rotation can be minimal, to allow free rotation of breathing circuit interface 16.

In another embodiment, the connection between breathing circuit interface 16 and rigid portion 21 of the mask body 12 may be achieved by using a ball bearing arrangement or any other type bearing arrangement that allows a rotating motion of breathing circuit interface 16 with respect to mask body 12.

As discussed above, inner surface 23 of breathing circuit interface 16 is shaped and configured to engage detachably with outer surface 25 of entrainment valve assembly 200 by a friction-fit. In addition to allowing friction fit with entrainment valve assembly 200, inner surface 23 of breathing circuit interface 16 allows entrainment valve assembly 200 to be removed and interchangeably friction fitted with different, other types of conduits 18 through a similar friction fit, as will be described in more detail later. In an example embodiment, the diameter of first connector portion 230 is larger than the diameter of second connector portion 232 of entrainment valve assembly 200 to prevent the wrong end of valve assembly 200 from being connected with interface 16.

Continuing to refer to the cross-sectional views of FIGS. 6 and 7, entrainment valve assembly 200 includes a valve member 208. Valve member 208 is connected to tubular member 201 at connection region 248 thereof by means of a recess 250, a barb 526 and a stop member 528 provided in the valve member 208. A rib 252 (FIG. 7), located on the lower portion of bent tubular region 233 of entrainment valve assembly 200, has an outer surface thereof that is received in recess 250 so as to clamp connecting region 248 against an annular region 253.

Valve member 208 includes a sealing portion 520, having a relatively thin, flat, oval configuration. Sealing portion 520 is made of a flexible material and thus is capable of bending upwardly (as shown in the dashed lines in FIG. 7) in response to pressurized gas being forced into primary inlet 202. The upward bending continues until an upper surface 522 of sealing portion 520 engages an annular lip 235 at the end of a cylindrical wall 254 protruding into tubular body 201 and defining secondary inlet 204. The direction of travel of sealing portion 520 from its rest position to the upper bent portion is shown by arrow A in FIG. 7. In this upper bent portion, the sealing engagement of upper surface 522 of valve member 208 with annular lip 235 causes secondary inlet 204 to be sealed so that pressurized gas provided into primary inlet 202 does not escape through secondary inlet 204.

It should be noted that where gas is not being provided to the pediatric patient through primary inlet 202 (e.g., pressure/flow generating system 42 connected with primary inlet 202 is not operating), secondary inlet 204 may serve as both an inlet passage of atmospheric air provided to the pediatric patient during inhalation and an outlet passage for exhalation. In this instance, sealing portion 520 may remain at its at rest position, wherein it forms a seal with an upper surface 259 of annular flange 253, as shown in FIG. 7.

Valve member 208 can be made from rubber, latex, silicone, or any other elastomeric material as would be appreciated by one skilled in the art.

As can be appreciated most readily from FIGS. 2 and 4, exhalation grooves 258 form a passage between exterior surface 25 of tubular portion 201 and interior cylindrical surface 23 of breathing circuit interface 16. In one embodiment, exhalation grooves 258 are provided on opposite lateral sides of exterior surface 25 of tubular portion 201. In another embodiment, exhalation grooves may be provided on inner surface 23 of breathing circuit interface 16 rather than on body 201. In addition, as shown as dashed lines in FIG. 7, in another embodiment they may alternatively, or also, be located at the top portion of exterior surface 25 of body 201. When the pediatric patient inhales, a very small fraction of gas may be drawn from atmosphere through exhalation grooves 258. However, by and large, the pressurized gas forced into primary inlet 202 will create higher pressure within body 201 than the atmospheric pressure, so that air is mostly forced outwardly through exhalation passages 258 (rather than inwardly), even during inhalation. Moreover, as the pediatric patient exhales, the exhaled gas impacts the centrally incoming airflow through body 201 and is thus forced to mushroom radially outwardly resulting in a circular flow pattern that effectively flushes the exhaled gas, and is thus generally directed toward and through peripheral exhalation grooves 258 to atmosphere.

The removable and replaceable conduits 18 enable mask assembly 10 to be functional for different uses, simply by employing the conduit 18 of choice. Although shown with a conduit 18 that is adapted to accommodate entrainment valve assembly 200, it is to be appreciated that the present invention contemplates that other conduits 18 may be employed with mask assembly 10 without varying from the scope of the present invention. Some non-limiting examples of other types of conduits 18 that can be used interchangeably with mask assembly 10 described above include: conduit with a bronchoscope port to permit the care giver to perform a bronchoscopy procedure with mask on; conduit with aerosol generator adapter to deliver medication during NIV; conduit with MDI port to deliver medication using a "Metered Dose Inhaler"; conduit with port to accommodate a CPAP relief valve; conduit with CO₂ sensor capabilities to monitor pediatric patient; conduit with Volumetric CO₂ sensor capabilities to monitor pediatric patient VCO₂; conduit that entrains Heliox or other specialty gases; conduit that adds moisture to inhaled gas; conduit that includes an HME (Heat moisture exchanger); conduit that incorporates "nano" sensors for a variety of clinical monitoring capabilities; conduit with Filtered Exhalation, which is useful in pandemic situations like SARS; conduit that enhances the patient's ability to "Speak with Mask On"; conduit that accommodates a NG feeding tube; conduit that reduces/control CO₂ re-breathing; conduit that aids in secretion clearance; conduit with Standard Elbow; conduit that can be used on a wide range of mask types, such as full (nasal/oral), nasal only, cannula, pillows, or total or Helmet type of masks.

It should be appreciated, that the above listed conduit configurations provide non-limiting examples of different types, configurations and/or constructions of conduits that can be provided. It should be appreciated that, while these conduits may all be provided with an elbow shaped tubular body, other tubular shapes (such as a straight tubular configuration) may alternatively be provided.

Other embodiments in accordance with the present invention are contemplated in which the connection between conduit 18 and breathing circuit interface 16 is not a friction fit, but may be achieved by virtue of other types of connections such as, for example, without limitation, a quarter-turn type connection, a snap fit, or any other locking mechanism that provides a detachable connection between conduit 18 and breathing circuit interface 16.

In yet another embodiment, first connector portion 230 of conduit 18 may itself be provided with a swivel coupling, similar to breathing circuit interface 16, rather than such structure being provided as part of the mask. In such case, the swivel coupling of the elbow can be connected directly to a non-swiveled portion (e.g., an outwardly projecting cylindrical configuration) surrounding opening 13 in rigid portion 21 of mask body 12.

In yet another embodiment, no swivel coupling is provided. Rather, a direct connection between the tubular body (e.g., 201) is provided with a correspondingly shaped portion of rigid portion 21 of the mask. In this embodiment, some rotation of conduits 18 may nevertheless be accommodated via direct sliding friction at the friction fit connection between rigid portion 21 and the tubular body. However, it is further contemplated that other, non-rotational connections may also be provided and will still enable the modularity of design contemplated herein.

In one aspect of the invention, a mask assembly kit is provided. The kit assembly includes mask body 12, with or without rotatable interface 16, and at least two conduits 18 of different types to enable mask body 12 to provide different functionality simply by changing conduit types. For example, a standard (valveless) elbow can be provided as one conduit, and the entrainment valve assembly 200 can be provided as another conduit. More than two conduits may be provided, and more than one mask may be provided, although each of the masks will have a common configuration, while the conduits will have at least two different configurations that fit the mask body. Additionally, an embodiment of the kit includes a plurality of headgear assemblies 11, with at least one headgear assembly being a color indicative of a male pediatric patient and another headgear assembly being a color indicative of a female patient, as discussed in greater detail below.

Referring to FIGS. 6, 8 and 9, details of flexible peripheral seal structure 20 will now be discussed. As best seen in FIG. 6, flexible peripheral seal structure 20 may have a generally rectangular channel shaped cross-sectional configuration with three sides 504, 506 and 508. Flexible peripheral seal structure 20 may be attached to mask body 12 at side 504. An edge 500 of rigid portion 21 of mask body 12 engages with an opening 502 located in side 504 of flexible peripheral seal structure 20, such that a layered connection is formed. The parts are then adhered in place, through an adhesive connection, an ultrasonic weld connection, a riveted, a pinned connection, or any other type of suitable connection as would be appreciated by one skilled in the art. Other embodiments are contemplated in which there is no overlap, such as by attaching rigid portion 21 and flexible peripheral seal structure 20 with their edges end to end (e.g., by an adhesive connection). Side 506 is located between side 504 and side 508, providing a gap between sides 504 and 508. This gap may provide flexibility to flexible peripheral seal structure 20, as it conforms to the face of the pediatric patient 27. The corners of flexible peripheral seal structure 20 may be generally rounded. The length of sides 508 and 506 may vary along the periphery of seal structure 20 so as to provide a conforming sealing engagement of mask body 12 with the face of pediatric patient 27.

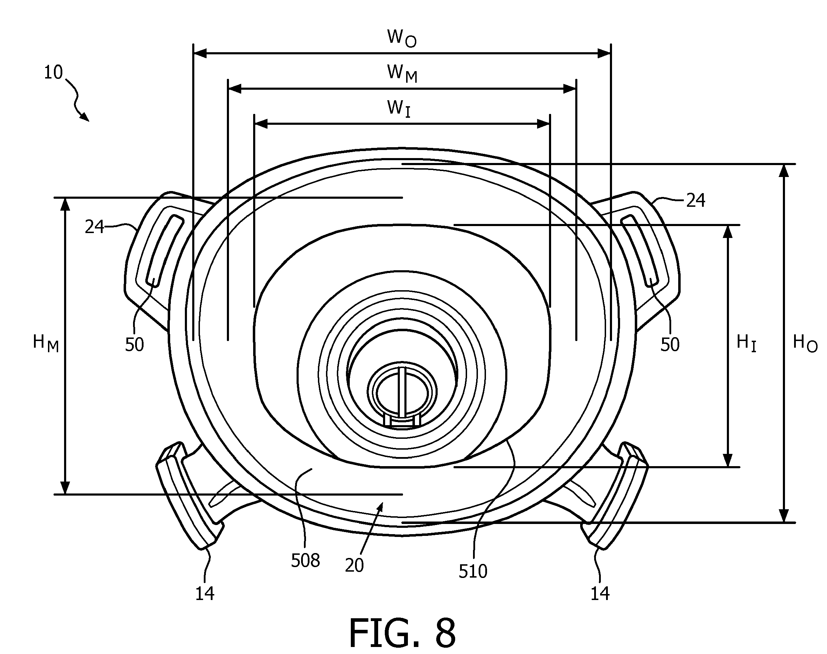

More particularly, as shown in the elevation view of FIG. 8, side 508 of peripheral seal structure 20 has a general overall height H_{O} and an overall width W_{O} and includes an opening, generally indicated at 510, that is particularly adapted to receive the pediatric patient's face therein, and thus provide for side 508 to sealingly engage the face of pediatric patient 27 about the edges of the patient's face. In order to accomplish such fitment, opening 510 is dimensioned having a height H_{I} and a width W_{I} proportioned specifically to a pediatric patients face. Side 508 is generally designed such that, at minimum, a seal is created about the pediatric patient's face at or about the midpoints between opening 510 and the outer edge of side 508. The general height and width of such minimum sealing region is shown in FIG. 8 by the dimensions H_{M} and W_{M}, respectively.

In an example embodiment, the ratio of height H_{I} to width W_{I} is about 0.83, in contrast to a typical ratio of about 1.0 which would be necessary to accommodate an adult patient's face. In other embodiments, the ratio of height H_{I} to width W_{I} is in the range of about 0.83 to about 0.87. In an example embodiment, opening 510 has a height H_{I} of about 60.5mm and a width W_{I} of about 73.0mm, and thus a height to width ratio of about 0.83. In the same example embodiment, side 508 also has an overall height H_{O} of about 87.9mm, an overall width of about 100.5mm, and intermediate dimensions H_{M},W_{M} of 74.8mm and 86.9mm, respectively.

In an example embodiment, the size of the face mask is embossed on the lower end portion of flexible peripheral seal structure 20, as shown by 600 in FIG. 9.

Flexible peripheral seal structure 20 may be made of a relatively soft and/or flexible material so that flexible peripheral seal structure 20 conforms to the shape of a pediatric patient's face when held against it. In an example embodiment, a material having a 20 durometer (as compared to a 40 durometer material commonly employed in adult applications) was employed. Flexible peripheral seal structure 20 may be made of, for example, silicone, an elastomeric material or any other suitable shape conforming material as will be appreciated by one skilled in the art. Different regions of the flexible peripheral seal structure 20 around the perimeter of mask body 12 may have different cross-sectional configurations. Various other flexible peripheral seal structure 20 configurations will become apparent to those skilled in the art. Rigid portion 21 of mask body 12, in one embodiment, is made of a relatively more rigid material than flexible peripheral seal structure 20. For example, mask body 12 may be made from polycarbonate, or other suitable material.

Mask body 12 may be formed by a two-step insert molding process. For example, rigid portion 21 may be molded first and then inserted into a second mold for flexible peripheral flexible peripheral seal structure 20, which is injection molded to form around and/or into rigid portion 21.

In an example embodiment, mask assembly 10 includes a mask body 12 having a part volume of 17.02 cm³ and a seal structure 20 having a part volume of 34.11 cm³.

In one embodiment, headgear assembly 11 that is used to mount mask body 12 to the head of pediatric patient 27 takes the form of a bonnet having straps. However, any structure that secures mask body 12 to the head of a pediatric patient can be used. In one embodiment, headgear assembly 11 is provided in three different colors, a shade of pink for girls, a shade of blue for boys, and a gender neutral color (e.g., yellow). In the illustrated embodiment as shown in FIGS. 1A and 1B, an end portion (not numbered) of each of two headgear straps 46 (only one shown in FIG. 1B) is threaded through elongated opening 50 of headgear retaining tab 24, and the end portion (not numbered) of lower headgear straps 40 are threaded through the elongated opening 130 of the headgear attachment clip 14. In one embodiment, the end portions comprise hook material and is bent back into engagement with the adjoining surface formed of loop material on the straps so as to form a hook and loop (or VELCRO™) type connection. It is to be appreciated, however, that there are numerous other ways for securing the end portion of the headgear strap to itself or to the headgear attachment clip 14 and/or to the headgear attachment tab 24, such as a snap connection, buckle, or locking clamp, as non-limiting examples. Headgear 11 is adjustable, as straps 44,46 can be pulled further through the opening 50 of the headgear retaining tab 24 or the elongated opening of headgear attachment clip 14 to accommodate smaller diameter head sizes.

In addition, in another embodiment, a more permanent attachment of the end portion of headgear straps 44,46 to headgear strap retaining tabs 24 or headgear attachment clips 14 may be provided. For example, once headgear assembly 11 has been fitted to the pediatric patient 27 by adjusting straps 44,46 to the desired lengths, the free ends of straps 44,46 can be permanently fixed back onto straps 44,46, such as by gluing, sewing, or riveting the overlapping straps together. Straps 44,46 of headgear assembly 11 may be elastic or inelastic, and may extend around the back of the head of the pediatric patient 27 to secure mask body 12 on the pediatric patient 27, with the flexible peripheral seal structure 20 in sealing engagement with the pediatric patient's face.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A mask assembly (10) for use in a system delivering a flow of a treatment gas to the airway of a pediatric patient, the mask assembly comprising:
a mask body (12) having an opening (13) adapted to receive a treatment gas supply; and
a flexible peripheral seal structure (20) having a first side (504) coupled to the mask body and an opposite second side (508) including an opening (510) adapted to receive the face of the pediatric patient such that the second side sealingly engages the perimeter of the pediatric patient's face, wherein the opening is defined by a height (H) and a width (W), and wherein the ratio of the height to width of the opening is in the range of 0.83 to 0.87.

2. The pediatric mask assembly of claim 1 wherein the ratio of the height to width of the opening is 0.83.

3. The pediatric mask assembly of claim 1 wherein the opening has a height of about 60.5mm and a width of about 73.0mm.

4. The pediatric mask assembly of claim 1 further comprising a headgear assembly (11) selectively coupled to the mask body.

5. The pediatric mask assembly of claim 4 wherein the headgear assembly comprises a bonnet-style headgear assembly.

6. The pediatric mask assembly of claim 5 wherein the headgear assembly comprises a color indicative of the gender of the pediatric patient for which the assembly is intended.

7. The pediatric mask assembly of claim 6 wherein the headgear assembly is one of: a color of blue to indicate a male pediatric patient or a color of pink to indicate a female patient.

8. A system for delivering treatment gas to the airway of a pediatric patient, the system comprising:
a pressure/flow generating system (42);
a tubing element (40) having a first end coupled to the pressure /flow generating system and an opposite second end;
the mask assembly (10) of claim 1, further comprising:
a headgear assembly (11) selectively coupled to the mask body (12), wherein the opening (13) of the mask body (12) is coupled to the second end of the tubing element (40) and adapted to receive a treatment gas supply from the pressure/flow generating system (42) via the tubing element (40).

9. The system of claim 8 wherein the opening the second side of the flexible peripheral seal structure has a height of about 60.5mm and a width of about 73mm.

10. The system of claim 8 wherein the headgear assembly comprises a bonnet-style headgear assembly.

11. The system of claim 10 wherein the headgear assembly comprises a color indicative of the gender of the pediatric patient for which the assembly is intended.

12. The system of claim 11 wherein the headgear assembly is one of: a color of blue to indicate a male pediatric patient or a color of pink to indicate a female patient.

13. A kit comprising:
the mask assembly (10) of claim 1 for use in a system delivering a flow of a treatment gas to the airway of a pediatric patient, the mask assembly further comprising:
a plurality of headgear assemblies, each headgear assembly being selectably coupleable to the mask assembly, wherein at least one headgear assembly comprises a color adapted to indicate a male patient and wherein at least another one headgear assembly comprises a color adapted to indicate a female patient.

14. The kit of claim 13 wherein each headgear assembly comprises a bonnet-style headgear assembly.

15. The kit of claim 14 wherein the color adapted to indicate a male patient comprises a shade of blue and wherein the color adapted to indicate a female patient comprises a shade of pink.

## Patentansprüche

1. Maskenbaugruppe (10) zur Verwendung in einem System zur Abgabe einer Behandlungsgasströmung an die Atemwege eines pädiatrischen Patienten, wobei die Maskenbaugruppe Folgendes umfasst:
einen Maskenkörper (12) mit einer Öffnung (13), die dafür ausgelegt ist, eine Behandlungsgaszuführung aufzunehmen; und
eine biegsame Umfangsabdichtungsstruktur (20) mit einer ersten Seite (504), die mit dem Maskenkörper gekoppelt ist, und einer gegenüberliegenden zweiten Seite (508), welche eine Öffnung (510) umfasst, die dafür ausgelegt ist, das Gesicht des pädiatrischen Patienten derartig aufzunehmen, dass die zweite Seite abdichtend in den Umfang des Gesichts des pädiatrischen Patienten eingreift, wobei die Öffnung durch eine Höhe (H) und eine Breite (W) definiert ist und wobei das Verhältnis von der Höhe zur Breite der Öffnung im Bereich von 0,83 bis 0,87 liegt.

2. Pädiatrische Maskenbaugruppe nach Anspruch 1, wobei das Verhältnis von der Höhe zur Breite der Öffnung 0,83 beträgt.

3. Pädiatrische Maskenbaugruppe nach Anspruch 1, wobei die Öffnung eine Höhe von etwa 60,5 mm und eine Breite von etwa 73,0 mm hat.

4. Pädiatrische Maskenbaugruppe nach Anspruch 1, weiterhin umfassend eine Kopfgeschirrbaugruppe (11), die selektiv mit dem Maskenkörper gekoppelt ist.

5. Pädiatrische Maskenbaugruppe nach Anspruch 4, wobei die Kopfgeschirrbaugruppe eine haubenartige Kopfgeschirrbaugruppe umfasst.

6. Pädiatrische Maskenbaugruppe nach Anspruch 5, wobei die Kopfgeschirrbaugruppe eine Farbe umfasst, die das Geschlecht des pädiatrischen Patienten angibt, für den die Baugruppe vorgesehen ist.

7. Pädiatrische Maskenbaugruppe nach Anspruch 6, wobei die Kopfgeschirrbaugruppe entweder von der Farbe Blau ist, um einen männlichen pädiatrischen Patienten anzugeben, oder von der Farbe Rosa ist, um einen weiblichen Patienten anzugeben.

8. System zur Abgabe eines Behandlungsgases an die Atemwege eines pädiatrischen Patienten, wobei das System Folgendes umfasst.
ein Druck/Strömungs-Erzeugungssystem (42);
ein Schlauchelement (40) mit einem ersten Ende, das mit dem Druck/Strömungs-Erzeugungssystem gekoppelt ist, und einem gegenüberliegenden zweiten Ende;
die Maskenbaugruppe (10) nach Anspruch 1, die weiterhin Folgendes umfasst:
eine Kopfgeschirrbaugruppe (11), die selektiv mit dem Maskenkörper (12) gekoppelt ist, wobei die Öffnung (13) des Maskenkörpers (12) mit dem zweiten Ende des Schlauchelements (40) gekoppelt ist und dafür ausgelegt ist, über das Schlauchelement (40) eine Behandlungsgaszuführung von dem Druck/Strömungs-Erzeugungssystem (42) zu empfangen.

9. System nach Anspruch 8, wobei die Öffnung der zweiten Seite der biegsamen Umfangsabdichtungsstruktur eine Höhe von etwa 60,5 mm und eine Breite von etwa 73,0 mm hat.

10. System nach Anspruch 8, wobei die Kopfgeschirrbaugruppe eine haubenartige Kopfgeschirrbaugruppe umfasst.

11. System nach Anspruch 10, wobei die Kopfgeschirrbaugruppe eine Farbe umfasst, die das Geschlecht des pädiatrischen Patienten angibt, für den die Baugruppe vorgesehen ist.

12. System nach Anspruch 11, wobei die Kopfgeschirrbaugruppe entweder von der Farbe Blau ist, um einen männlichen pädiatrischen Patienten anzugeben, oder von der Farbe Rosa ist, um einen weiblichen Patienten anzugeben.

13. Kit, das Folgendes umfasst:
die Maskenbaugruppe (10) nach Anspruch 1 zur Verwendung in einem System zur Abgabe einer Behandlungsgasströmung an die Atemwege eines pädiatrischen Patienten, wobei die Maskenbaugruppe weiterhin Folgendes umfasst:
eine Vielzahl von Kopfgeschirrbaugruppen, wobei jede Kopfgeschirrbaugruppe auswählbar mit der Maskenbaugruppe koppelbar ist, wobei mindestens eine Kopfgeschirrbaugruppe eine Farbe umfasst, die dafür ausgelegt ist, einen männlichen Patienten anzugeben, und wobei mindestens eine Kopfgeschirrbaugruppe eine Farbe umfasst, die dafür ausgelegt ist, einen weiblichen Patienten anzugeben.

14. Kit nach Anspruch 13, wobei jede Kopfgeschirrbaugruppe eine haubenartige Kopfgeschirrbaugruppe umfasst.

15. Kit nach Anspruch 14, wobei die Farbe, die dafür ausgelegt ist, einen männlichen Patienten anzugeben, einen Blauton umfasst, und wobei die Farbe, die dafür ausgelegt ist, einen weiblichen Patienten anzugeben, einen Rosaton umfasst.

## Revendications

1. Ensemble masque (10) destiné à être utilisé dans un système distribuant un flux d'un gaz de traitement aux voies respiratoires d'un patient pédiatrique, l'ensemble masque comprenant :
un corps de masque (12) comportant une ouverture (13) conçue pour recevoir une alimentation en gaz de traitement ; et
une structure de joint périphérique souple (20) comportant un premier côté (504) couplé au corps de masque et un second côté opposé (508) comprenant une ouverture (510) conçue pour recevoir le visage du patient pédiatrique de telle sorte que le second côté vient en prise de manière étanche avec le périmètre du visage du patient pédiatrique, dans lequel l'ouverture est définie par une hauteur (H) et une largeur (W), et dans lequel le rapport de la hauteur à la largeur de l'ouverture est dans la plage de 0,83 à 0,87.

2. Ensemble masque pédiatrique selon la revendication 1, dans lequel le rapport de la hauteur à la largeur de l'ouverture est de 0,83.

3. Ensemble masque pédiatrique selon la revendication 1, dans lequel l'ouverture a une hauteur d'environ 60,5 mm et une largeur d'environ 73,0 mm.

4. Ensemble masque pédiatrique selon la revendication 1, comprenant en outre un ensemble casque (11) couplé de façon sélective au corps de masque.

5. Ensemble masque pédiatrique selon la revendication 4, dans lequel l'ensemble casque comprend un ensemble casque de style bonnet.

6. Ensemble masque pédiatrique selon la revendication 5, dans lequel l'ensemble casque comprend une couleur caractéristique du sexe du patient pédiatrique pour lequel l'ensemble est prévu.

7. Ensemble masque pédiatrique selon la revendication 6, dans lequel l'ensemble casque est d'une couleur parmi : une couleur bleue pour indiquer un patient pédiatrique garçon ou une couleur rose pour indiquer un patient fille.

8. Système de distribution d'un gaz de traitement aux voies respiratoires d'un patient pédiatrique, le système comprenant :
un système producteur de pression/flux (42) ;
un élément de tubage (40) dont une première extrémité est couplée au système producteur de pression/flux et une seconde extrémité opposée ;
l'ensemble masque (10) selon la revendication 1, comprenant en outre :
un ensemble casque (11) couplé de façon sélective au corps de masque (12), dans lequel l'ouverture (13) du corps de masque (12) est couplée à la seconde extrémité de l'élément de tubage (40) et conçue pour recevoir une alimentation en gaz de traitement provenant du système producteur de pression/flux (42) par l'intermédiaire de l'élément de tubage (40).

9. Système selon la revendication 8, dans lequel l'ouverture du second côté de la structure de joint périphérique souple a une hauteur d'environ 60,5 mm et une largeur d'environ 73 mm.

10. Système selon la revendication 8, dans lequel l'ensemble casque comprend un ensemble casque de style bonnet.

11. Système selon la revendication 10, dans lequel l'ensemble casque comprend une couleur caractéristique du sexe du patient pédiatrique pour lequel l'ensemble est prévu.

12. Système selon la revendication 11, dans lequel l'ensemble casque est d'une couleur parmi : une couleur bleue pour indiquer un patient pédiatrique garçon ou une couleur rose pour indiquer un patient fille.

13. Trousse comprenant :
l'ensemble masque (10) selon la revendication 1, destiné à être utilisé dans un système distribuant un flux d'un gaz de traitement aux voies respiratoires d'un patient pédiatrique, l'ensemble masque comprenant en outre :
une pluralité d'ensembles casques, chaque ensemble casque pouvant être couplé de façon sélectionnable à l'ensemble masque, dans laquelle au moins un ensemble casque comprend une couleur conçue pour indiquer un patient garçon et dans laquelle au moins un autre ensemble casque comprend une couleur conçue pour indiquer un patient fille.

14. Trousse selon la revendication 13, dans laquelle chaque ensemble casque comprend un ensemble casque de style bonnet.

15. Trousse selon la revendication 14, dans laquelle la couleur conçue pour indiquer un patient garçon comprend une nuance de bleu et dans laquelle la couleur conçue pour indiquer un patient fille comprend une nuance de rose.
